# EUROPÄISCHE PATENTSCHRIFT

(11) **EP 2 501 692 B1**
(45) Veröffentlichungstag und Bekanntmachung des Hinweises auf die Patenterteilung: **03.09.2014**
(21) Anmeldenummer: 10773338.8
(22) Anmeldetag: 06.11.2010
(51) Int. Cl.: C07D 401/12, C07C 257/20

(54) **VERFAHREN ZUR HERSTELLUNG VON DABIGATRAN ETEXILAT**
Method for producing dabigatran etexilate
Procédé de fabrication de dabigatran étexilate

(30) Priorität: 18.11.2009 EP 09176369
(43) Veröffentlichungstag der Anmeldung: 26.09.2012
(73) Patentinhaber: Boehringer Ingelheim International GmbH, 55216 Ingelheim am Rhein (DE)
(72) Erfinder: GNAD, Frieder, 55216 Ingelheim am Rhein (DE); DACH, Rolf, 55216 Ingelheim am Rhein (DE); HEDDESHEIMER, Ingo, 55216 Ingelheim am Rhein (DE); HEITGER, Helmut, 55216 Ingelheim am Rhein (DE); MEINECK, Siegfried, 55216 Ingelheim am Rhein (DE); MUELLER-BOETTICHER, Hermann, 55216 Ingelheim am Rhein (DE); SCHMITT, Stefan, 55216 Ingelheim am Rhein (DE)
(74) Vertreter: Simon, Elke Anna Maria
(86) Internationale Anmeldenummer: PCT/EP2010/066959
(87) Internationale Veröffentlichungsnummer: WO 2011/061080

(56) Entgegenhaltungen:
- WO-A-2006/000353
- WO-A-2007/071742
- US-B1- 6 358 960

## Beschreibung

### HINTERGRUND DER ERFINDUNG

Substituierte (4-Benzimidazol-2-ylmethylamino)-benzamidine, insbesondere Dabigatran Etexilat (CAS 593282-20-3), sind bereits aus der Internationalen Patentanmeldung WO 98/37075 als Wirkstoffe mit einer Thrombin-hemmenden und die Thrombinzeit verlängernden Wirkung offenbart werden, bekannt. Hauptindikationsgebiete der Verbindung der chemischen Formel I sind die postoperative Prophylaxe von tiefen Venenthrombosen und die Schlaganfallprophylaxe (prevention of stroke due to atrial fibrillation, kurz SPAF).

In WO 98/37075 wird vorgeschlagen die substituierten (4-Benzimidazol-2-ylmethylamino)-benzamidine durch Umsetzung entsprechend, substituierter (4-Benzimidazol-2-ylmethylamino)-benzonitrile mit Ammoniak herzustellen. Dieses Verfahren ist produktionstechnisch sehr aufwändig und führt zu einer hohen Belastung an zu entsorgenden Säuren (siehe auch WO 2007/071743, WO 2007/ 071742).

Nachstehend ist ein verbessertes Verfahren zur Herstellung von Dabigatran Etexilat sowie analoger Verbindungen davon, beschrieben. Durch die Umstellung auf neue Startmaterialien, die Anwendung von Phasentransferkatalyse und die Bildung des Benzimidazols ohne Verwendung von Kupplungsreagenzien wird eine deutlich effizientere Synthese von Dabigatran Etexilat erzielt. Die hohe Selektivität bei der Kupplung der Intermediate (Stufe 2) trägt dabei im Wesentlichen zur Wirtschaftlichkeit der neuen Syntheseroute bei.

### DETAILLIERTE BESCHREIBUNG DER ERFINDUNG

Die vorliegende Erfindung beschreibt ein Verfahren zur Herstellung von Verbindungen der Formel **7**: 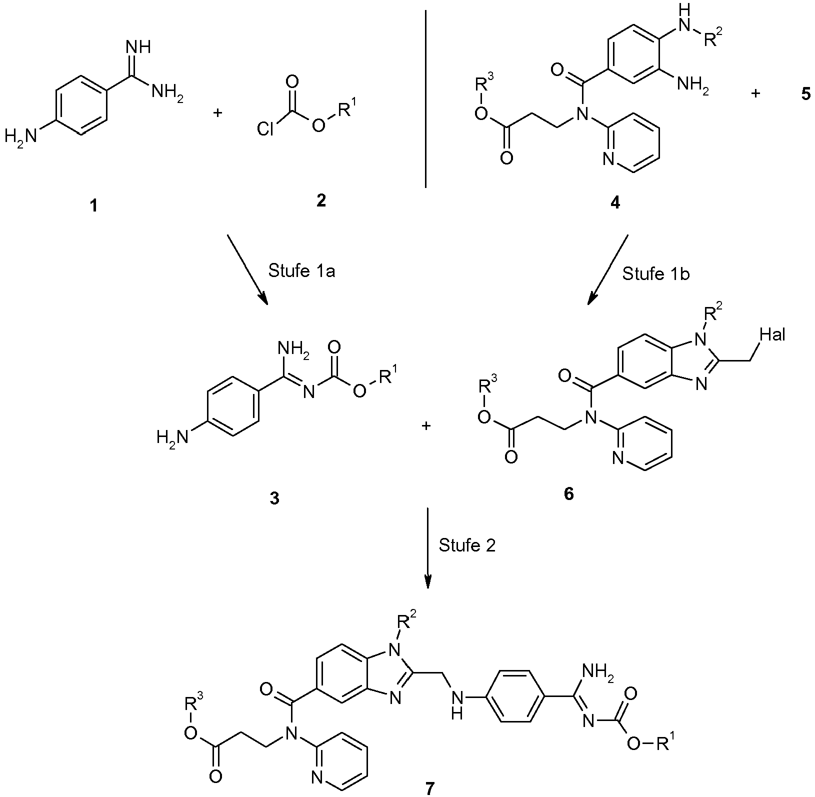 worin R¹, R² und R³ hier und im Folgenden jeweils unabhängig voneinander C₁₋₆-alkyl bedeuten und Hal = Chlor oder Brom, bevorzugt Chlor bedeutet, für **5** können erfindungsgemäß Halogenessigsäureanhydrid **5b-1,** Halogenessigsäure **5b-2**, Halogenessigsäureorthoester **5b-3** oder Halogenacetylchlorid **5b-4** eingesetzt werden, bevorzugt sind für **5** Halogenessigsäureanhydrid **5b-1** oder Halogenessigsäureorthoester **5b-3** eingesetzt werden.

Bevorzugt bedeuten R¹, R² und R³ hier und im Folgenden jeweils unabhängig voneinander Methyl, Ethyl, Propyl, Butyl oder Hexyl, besonders bevorzugt Methyl, Ethyl oder Hexyl, insbesondere bedeuten R¹ = Hexyl; R² = Methyl und R³ = Ethyl.

In Stufe 1a reagieren p-Aminobenzamidin **1** und C₁₋₆-alkylchloroformiat **2** zum Intermediat **3** (4-Aminobenzamidin-C₁₋₆-alkyl-carbamat).

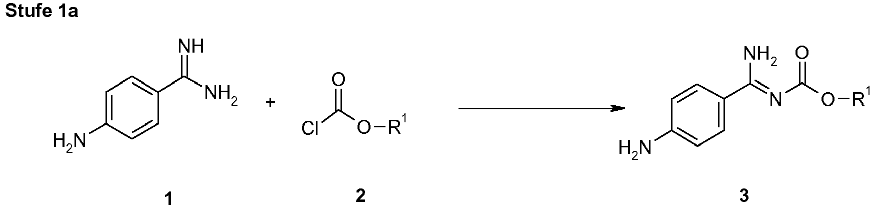

Hierfür wird Aminobenzamidin **1**, bevorzugt als Hydrochlorid, besonders bevorzugt als Dihydrochlorid in einem polaren Lösungsmittel ausgewählt aus der Gruppe bestehend aus Aceton, Ethylacetat und Butylacetat, bevorzugt Aceton, auf weniger als 40°C, bevorzugt 10 bis 35°C, besonders bevorzugt 15 bis 25°C, insbesondere 18 bis 22°C abgekühlt. Anschließend wird NaOH oder eine Vergleichbare Base und ein Chloroformiat **2** (R¹ = C₁₋₆-alkyl) zugegeben. Im Anschluss an die Reaktionszeit von etwa 5 bis 30 min, bevorzugt 10 bis 20 min werden die Phasen getrennt.

Das Gemisch wird eingeengt und mit einem polaren Lösungsmittel ausgewählt aus der Gruppe bestehend aus Butylacetat und Ethylacetat, bevorzugt Butylacetat, verdünnt und mit Wasser durch Extraktion gereinigt.

Anschließend wird das Produkt mit einer Säure **S** ausgewählt aus der Gruppe bestehend aus Salzsäure, Oxalsäure und Methansulfonsäure, bevorzugt Salzsäure, gefällt und gegebenenfalls mit organischen Lösungsmitteln ausgewählt aus der Gruppe bestehend aus Aceton, Butylacetat und Ethylacetat oder Gemischen davon, bevorzugt ein Gemisch aus Aceton und Butylacetat gewaschen. Das bevorzugte Mischungsverhältnis von Aceton zu Butylacetat ist 1:1. Man erhält die Verbindung **3** als das entsprechende Salz der oben genannten Säure **S**.

In einer parallelen Stufe 1 b wird die Verbindung **4** mit Verbindung **5** zu Intermediat **6** umgesetzt.

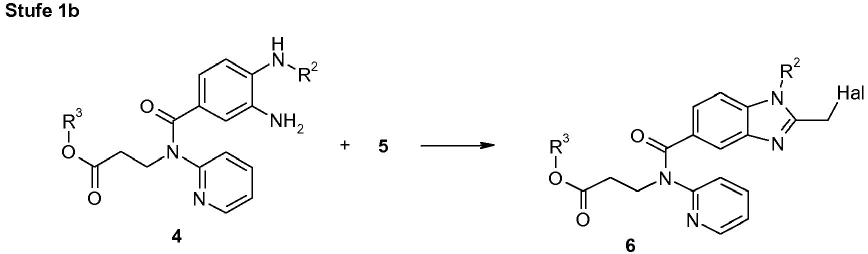

Die Synthese von Benzimidazolen mit durch Kupplungsreagenzien aktivierten Carbonsäuren oder Säurechloriden ist bekannt, bislang jedoch nicht mit α-Monochloressigsäureanhydriden beschrieben. Dabei können für **5** erfindungsgemäß folgende Verbindungen eingesetzt werden:
- Variante 1 b-1: Halogenessigsäureanhydrid **5b-1**;
- Variante 1 b-2: Halogenessigsäure **5b-2;**
- Variante 1b-3: Halogenessigsäureorthoester **5b-3**, bevorzugt der Formel Hal-CH₃-C(OR⁴)₃, (R⁴ = unabhängig voneinander C₁₋₆-alkyl, bevorzugt unabhängig voneinander Methyl oder Ethyl), beispielhaft sei 2,2,2-Triethoxy-chlorethan genannt;
- Variante 1b-4: Halogenacetylchlorid **5b-4**.

Worin Halogen (Hal) = Brom oder Chlor bedeutet, bevorzugt bedeutet es Chlor. Bei allen Varianten 1 b-1, 1 b-2, 1 b-3 und 1 b-4 ist die vorherige Isolierung des Diamins **4** (R^{2/3} = C₁₋₆-alkyl) nicht notwendig. Es kann ebenfalls die Produktlösung aus einer Reduktionsreaktion der Nitroverbindung wie sie im Stand der Technik beschrieben ist (siehe WO 98/37075, WO 2007/071743, WO 2007/ 071742) umgesetzt werden.

Für Variante 1 b-1 wird die Verbindung **4** in gekühltem Lösungsmittel suspendiert, worin das Lösungsmittel ausgewählt ist aus der Gruppe bestehend aus Ethylacetat, Butylacetat und Tetrahydrofuran, bevorzugt Ethylacetat, und die Temperatur weniger als 50°C, bevorzugt 0 bis 30°C, besonders bevorzugt 5 bis 25°C, insbesondere 18 bis 22°C beträgt. Der Suspension wird z.B. Chloressigsäureanhydrid **5b-1**' zugeben und im Anschluss auf 50 bis 80°C, bevorzugt auf 55 bis 75°C, besonders bevorzugt auf 60 bis 70°C, insbesondere auf 65°C erhitzt. Nach einem Zeitraum von 1 bis 6 Stunden, bevorzugt 1 bis 4 Stunden , besonders bevorzugt 1 bis 3 Stunden, insbesondere 2 Stunden wird bei einer Temperatur von 20 bis 60°C, bevorzugt 30 bis 50°C, besonders bevorzugt auf 35 bis 45°C, insbesondere 40°C eine schwache Base ausgewählt aus der Gruppe bestehend aus Kaliumcarbonat, Natriumcarbonat und Natriumhydrogencarbonat, bevorzugt Kaliumcarbonat, zugegeben und für weitere 30 bis 60 min, bevorzugt 40 bis 50 min, besonders bevorzugt 45 min gerührt. Nach Filtration wird das Filtrat mit einem Lösungsmittel ausgewählt ist aus der Gruppe bestehend aus Ethylacetat, Butylacetat und Tetrahydrofuran, bevorzugt Ethylacetat gewaschen, eingeengt und bei einer Temperatur von 25 bis 65°C, bevorzugt 35 bis 55°C, besonders bevorzugt auf 40 bis 50°C, insbesondere 45°C durch Zugabe eines weiteren Lösungsmittels ausgewählt ist aus der Gruppe bestehend aus MTBE und Tetrahydrofuran, bevorzugt MTBE, gefällt. Durch Abkühlen des Gemisches lässt sich die Fällung verbessern. Das so erhaltene Produkt wird mit organischen Lösungsmitteln ausgewählt aus der Gruppe bestehend aus Ethylacetat, Butylacetat, MTBE und Tetrahydrofuran oder Gemischen davon, bevorzugt ein Gemisch aus Ethylacetat und MTBE, gewaschen. Nach Trocknung des Filterkuchens erhält man Produkt **6**.

Variante 1b-2: Verbindung 4 wird in Toluol mit Molekularsieb (4 Angström) und z.B. Chloressigsäure versetzt. Die Mischung wird auf max. 60°C, bevorzugt 30 bis 55°C, besonders bevorzugt auf 35 bis 55°C, insbesondere 50°C erwärmt und gerührt. Nach einem Zeitraum von 1 bis 8 Stunden, bevorzugt 1 bis 6 Stunden, besonders bevorzugt 1 bis 4 Stunden, insbesondere 3 Stunden wird die Mischung auf max. 20 °C abgekühlt und das Produkt gefällt. Das so erhaltene Produkt wird mit Toluol gewaschen. Nach Trocknung des Filterkuchens erhält man Produkt **6**.

Für Variante 1 b-3 wird die Verbindung **4** in polarem Lösungsmittel ausgewählt aus der Gruppe bestehend aus Ethylacetat, Butylacetat und Tetrahydrofuran, bevorzugt Ethylacetat suspendiert, z.B. Chloressigsäureorthoester **5b-3'** sowie optional p-Toluolsulphonsäure zugeben und im Anschluss auf 40 bis 80°C, bevorzugt auf 50 bis 70°C, besonders bevorzugt auf 55 bis 65°C, insbesondere auf 60°C erhitzt. Nach einem Zeitraum von 1 bis 6 Stunden, bevorzugt 2 bis 5 Stunden, besonders bevorzugt 2.5 bis 3.5 Stunden, insbesondere 3 Stunden wird das Reaktionsgemisch im Vakuum eingeengt, der Rückstand mit einem Lösungsmittel ausgewählt aus der Gruppe bestehend aus MTBE und Tetrahydrofuran, bevorzugt MTBE gefällt. Durch Abkühlen des Gemisches lässt sich die Fällung verbessern. Das so erhaltene Produkt wird mit organischen Lösungsmitteln ausgewählt aus der Gruppe bestehend aus Ethylacetat, MTBE und Tetrahydrofuran oder Gemischen davon, bevorzugt ein Gemisch von Ethylacetat und MTBE gewaschen. Nach Trocknung des Filterkuchens erhält man Produkt **6**.

Für Variante 1 b-4 wird die Verbindung **4** in einem Lösungsmittel ausgewählt aus der Gruppe bestehend aus Ethylacetat, THF und Dioxan suspendiert, z.B. Chloracetylchlorid **5b-4'** innerhalb 3h bei 50°C zugeben und im Anschluss mit NaOH oder einer vergleichbaren Base alkalisch gestellt. Danach wird die Wasserphase abgetrennt und die organische Phase eingeengt, mit einem polaren Lösungsmittel ausgewählt und aus der Gruppe bestehend aus Butylacetat und Ethylacetat aufgenommen, die Phasen getrennt und die organische Phase erneut eingeengt. Der Rückstand wird mit einem Lösungsmittel ausgewählt aus der Gruppe bestehend aus MTBE und Tetrahydrofuran gefällt. Durch Abkühlen des Gemisches lässt sich die Fällung verbessern. Das so erhaltene Produkt wird mit organischen Lösungsmitteln ausgewählt aus der Gruppe bestehend aus Butylacetat, Ethylacetat, MTBE und Tetrahydrofuran oder Gemischen davon gewaschen. Nach Trocknung des Filterkuchens erhält man Produkt **6.**

Die Intermediate **3** und **6** werden in Stufe 2 unter Phasentransferkatalyse und Aktivierung mit lodid zur Verbindung **7** umgesetzt.

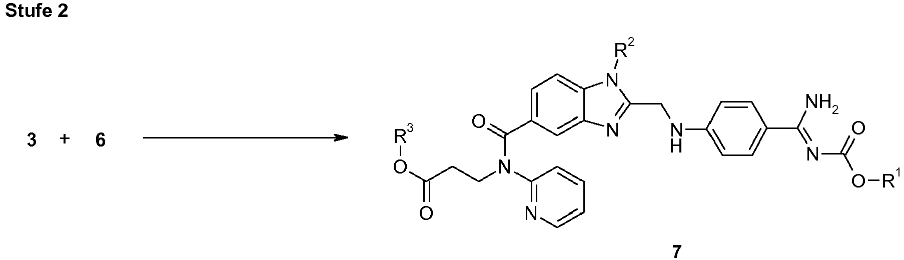

Die Kupplung von Alkylchloriden und Amidinen unter Katalyse von lodidionen ist bekannt, jedoch war bislang keine hohe Selektivität dieser Kupplungsreaktion berichtet, weshalb auch die Synthesen aus dem Stand der Technik auf doppelt geschützte Amidine auswichen. Überraschenderweise lässt sich nach dem im Folgenden beschriebenen Verfahren eine Kupplungsreaktion mit einfach geschütztem p-Aminobenzamidin (**3**) mit hoher Regioselektivität (>99.7%) durchführen.

Dafür wird Verbindung **3**, sowie eine Base ausgewählt aus der Gruppe bestehend aus NaOH, Kaliumcarbonat und Natriumcarbonat, bevorzugt NaOH, in einem Gemisch aus einem organischen Lösungsmittel ausgewählt aus der Gruppe bestehend aus Toluol, Tetrahydrofuran, 2-Methyltetrahydrofuran, Butylacetat und Ethylacetat, bevorzugt Butyl-acetat und Wasser vorgelegt und auf 30 bis 65°C, bevorzugt auf 40 bis 60°C, besonders bevorzugt auf 45 bis 55°C, insbesondere auf 50°C erwärmt. Danach werden die Phasen getrennt und optional die organische Phase ein weiteres Mal mit Wasser extrahiert.

Die organische Phase wird mit Verbindung **6**, sowie Natriumiodid, Natriumhydrogencarbonat, Tetrabutylammoniumiodid, in Cyclohexan und Wasser versetzt und im Anschluss auf 30 bis 60°C, bevorzugt auf 35 bis 50°C, besonders bevorzugt auf 35 bis 45°C, insbesondere auf 40°C erhitzt. Nach einem Zeitraum von 1 bis 6 Stunden, bevorzugt 1 bis 4 Stunden, besonders bevorzugt 1 bis 3 Stunden, insbesondere 2 Stunden wird das Cyclohexan abdestiliert und Butylacetat zugegeben und abermals auf 50 bis 90°C, bevorzugt auf 60 bis 80°C, besonders bevorzugt auf 65 bis 75°C, insbesondere auf 70°C über einen Zeitraum von 1 bis 6 Stunden, bevorzugt 1 bis 4 Stunden , besonders bevorzugt 1 bis 3 Stunden, insbesondere 2 Stunden, erhitzt. Im Anschluss werden die Phasen getrennt und optional die organische Phase mit Wasser extrahiert. Die organische Phase wird eingeengt, abgekühlt und filtriert. Das so erhaltene Produkt wird mit organischen Lösungsmitteln ausgewählt aus der Gruppe bestehend aus Butylacetat und MTBE, bevorzugt Butylacetat oder Gemischen davon gewaschen. Nach Trocknung des Filterkuchens erhält man Produkt **7** (R^{1/2/3} = C₁₋₆-alkyl).

Die Selektivität und die Reaktionsgeschwindigkeit werden in erheblichem Maße durch das oben genannte Lösungsmittelsystem beeinflusst. Insbesondere bei Verwendung eines Zweiphasensystems aus Wasser und zwei unterschiedlich polaren org. Lösemitteln wie Butylacetat / Cyclohexan ist der Umsatz bei hervorragender Reaktionszeit und entsprechender Produktreinheit möglich.

Optional kann die Verbindung der Formel **7** in einer dritten Stufe zum Mesilat **8** analog dem Stand der Technik durch Umsetzung von **7** mit Methansulfonsäure **9** umgewandelt werden.

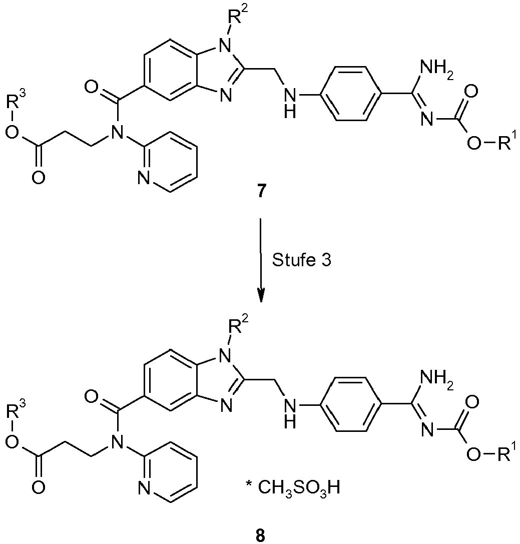

Ein weiterer Aspekt der Erfindung sind die neuen Zwischenprodukte des obigen Prozesses. Dies umfasst Verbindungen der Formel **3** 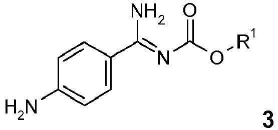 worin der Reste R¹ C₁₋₆-alkyl, bevorzugt Methyl, Ethyl, Propyl, Butyl oder Hexyl, besonders bevorzugt Methyl, Ethyl oder Hexyl, insbesondere n-Hexyl bedeutet.

Darüber hinaus sind Verbindungen der Formel **6** Gegenstand der Erfindung, 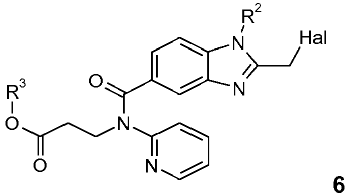 worin die Reste R² und R³ jeweils unabhängig voneinander C₁₋₆-alkyl, bevorzugt unabhängig voneinander Methyl, Ethyl, Propyl, Butyl oder Hexyl, besonders bevorzugt Methyl, Ethyl oder Hexyl und insbesondere R² = Methyl und R³ = Ethyl bedeuten, sowie Hal die Bedeutung von Chlor oder Brom, bevorzugt Chlor hat.

### DEFINITIONEN

Unter dem Begriff "C₁₋₆-Alkyl" (auch soweit sie Bestandteil anderer Reste sind) werden verzweigte und unverzweigte Alkylgruppen mit 1 bis 6 Kohlenstoffatomen verstanden und unter dem Begriff "C₁₋₄-Alkyl" verzweigte und unverzweigte Alkylgruppen mit 1 bis 4 Kohlenstoffatomen verstanden. Bevorzugt sind Alkylgruppen mit 1 bis 4 Kohlenstoffatome. Beispielsweise werden hierfür genannt: Methyl, Ethyl, *n*-Propyl, *iso*-Propyl, *n*-Butyl, *iso-*Butyl, *sec*-Butyl, *tert*-Butyl, *n*-Pentyl, *iso*-Pentyl, *neo*-Pentyl oder *n*-Hexyl. Gegebenenfalls werden für die vorstehend genannten Gruppen auch die Abkürzungen Me, Et, *n*-Pr, *i*-Pr, *n*-Bu, *i*-Bu, *t*-Bu, etc. verwendet. Sofern nicht anders beschrieben, umfassen die Definitionen Propyl, Butyl, Pentyl und Hexyl alle denkbaren isomeren Formen der jeweiligen Reste. So umfasst beispielsweise Propyl *n*-Propyl und *iso*-Propyl, Butyl umfasst *iso*-Butyl, sec-Butyl und *tert*-Butyl etc.

Unter einem "organischen Lösungsmittel" wird im Rahmen der Erfindung ein organsicher, niedermolekularer Stoff verstanden, der andere organische Stoffe auf physikalischem Wege zur Lösung bringen kann. Voraussetzung für die Eignung als Lösungsmittel ist, dass sich beim Lösungsvorgang weder der lösende noch der gelöste Stoff chemisch verändern, dass also die Komponenten der Lösung durch physikalische Trennverfahren wie Destillation, Kristallisation, Sublimation, Verdunstung, Adsorption in der Originalgestalt wieder gewonnen werden können. Aus verschiedenen Gründen können nicht nur die reinen Lösungsmittel, sondern Gemische, die die Lösungseigenschaften vereinigen verwendet werden. Beispielsweise seinen genannt:
- Alkohole, bevorzugt Methanol, Ethanol, Propanol, Butanol, Octanol, Cyclohexanol;
- Glykole, bevorzugt Ethylenglykol, Diethylenglykol;
- Ether / Glykolether, bevorzugt Diethylether, tert-Butylmethylether, Dibutylether, Anisol, Dioxan, Tetrahydrofuran, Mono-, Di-, Tri-, Polyethylenglykolether;
- Ketone, bevorzugt Aceton, Butanon, Cyclohexanon;
- Ester, bevorzugt Essigsäureester, Glykolester;
- Amide u.a. Stickstoff-Verbindungen, bevorzugt Dimethylformamid, Pyridin, N-Methylpyrrolidon, Acetonitril;
- Schwefel-Verbindungen, bevorzugt Schwefelkohlenstoff, Dimethylsulfoxid, Sulfolan;
- Nitro-Verbindungen bevorzugt Nitrobenzol;
- Halogenkohlenwasserstoffe, bevorzugt Dichlormethan, Chloroform, Tetrachlormethan, Tri-, Tetrachlorethen, 1,2-Dichlorethan, Chlorfluorkohlenstoffe;
- aliphatische oder alicyclische Kohlenwasserstoffe, bevorzugt Benzine, Petrolether, Cyclohexan, Methylcyclohexan, Decalin, Terpen-L.; oder
- aromatische Kohlenwasserstoffe, bevorzugt Benzol, Toluol, o-Xylol, m-Xylol, p-Xylol; oder entsprechende Gemische davon.

### BEISPIELE

### STUFE 1A: SYNTHESE VON 4-AMINOBENZAMIDIN-N-HEXYLCARBAMAT

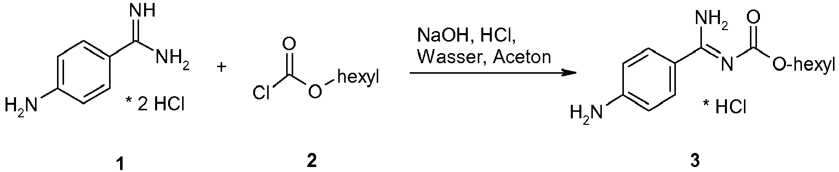

Aminobenzamidin * 2 HCl (21.2 g) wird in Aceton (150 ml) gelöst, auf 20°C temperiert und Natronlauge (80 ml, 4M) zugetropft. Bei 20°C wird n-Hexylchloroformiat (16.5 g) zudosiert. Nach Nachspülen mit Aceton (20 mL) werden weitere 15min bei 5-10°C gerührt. Im Anschluss werden die Phasen getrennt. Die organische Phase wird unter Vakuum eingeengt, mit Butylacetat (150 mL) verdünnt und erneut die Phasen getrennt. Es wird noch-mals mit Wasser (40 ML) extrahiert und mit Salzsäure (9, 84 mL, 32%ig) versetzt. Das Restwasser wird am Wasserabscheider abdestilliert und anschließend eingeengt. Die Suspension wird bei 45°C mit Aceton (150 mL) versetzt, auf 20°C abgekühlt und abgesaugt. Es wird mit einem Gemisch aus Butylacetat und Aceton (100 mL) gewaschen. Der Filterkuchen wird im Vakuum getrocknet und man erhält 29.2 g Produkt **3** (97.2 % d.Th).

### STUFE 1B: SYNTHESE VON 6 (β-ALANINE-N-[[1-METHYL-1 H-BENZIMIDAZOL-2-CHLORMETHYL]-5-CARBONYL]-N-2-PYRIDINYL-ETHYL ESTER):

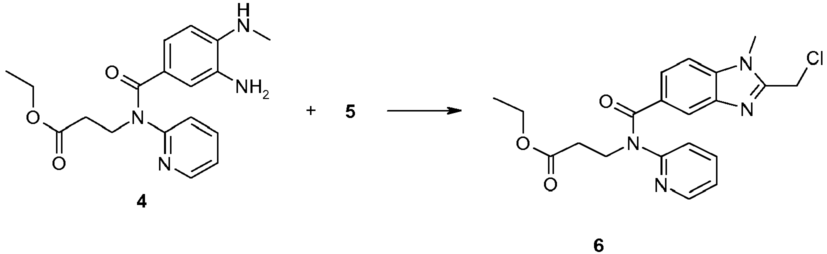

**5** kann je nach Synthesevariante Chloressigsäureanhydrid **5b-1',** Chloressigsäure **5b-2'** oder ein Chloressigsäureorthoester **5b-3'** oder Chloracetylchlorid **5b-4'** sein.

**Variante 1 b-1:** Verbindung **4** (28,0 g) wird in Ethylacetat (120 mL) bei 20°C suspendiert. Dann wird langsam ein Gemisch aus Ethylacetat (50 mL) und Chloressigsäureanhydrid **5b-1'** (14,5 g) bei 20°C zugeben und im Anschluss auf 65°C erhitzt. Nach 2h rühren wird bei 40°C Kaliumcarbonat (15,0 g) zugeben und nach 45min filtriert. Der Filterrückstand wird mit Ethylacetat (8,0 mL) gewaschen. Das Filtrat wird im Vakuum eingeengt und bei 45°C mit MTBE (150 mL) gefällt. Es wird auf -2°C abgekühlt und filtriert. Das Produkt wird mit einem Gemisch aus Ethylacetat und tert-Butylmethylether (MTBE) (50 mL) gewaschen. Der Filterkuchen wird im Vakuum getrocknet und man erhält 29.6 g Produkt **6**(90.3 % d.Th).

**Variante 1 b-2:** Verbindung 4 (2 g) wird in Toluol (20 mL) mit Molekularsieb (4A, 2 g) und Chloressigsäure (2,08 g)versetzt. Die Mischung wird auf 50°C erwärmt und gerührt. Nach einem Zeitraum von etwa 3 Stunden wird die Mischung auf unter 20 °C abgekühlt und das Produkt gefällt. Das so erhaltene Produkt wird mit Toluol gewaschen. Nach Trocknung des Filterkuchens erhält man Produkt **6** (30 % d. Th.)

**Variante 1 b-3:** Verbindung **4** (4,28 g) wird in Ethylacetat (26 mL) bei Raumtemperatur suspendiert und mit Chloressigsäureorthoester **5b-3'** (2,79 g) und p-Toluolsulphonsäure (0.02 g) versetzt und im Anschluss auf 60°C erhitzt. Nach 3h rühren wird das Reaktionsgemisch im Vakuum eingeengt, der Rückstand mit MTBE (25 mL) kristallisiert und filtriert. Das Produkt wird mit MTBE (25 mL) gewaschen. Der Filterkuchen wird im Vakuum getrocknet und man erhält 4,77 g Produkt **6** (95.2 % d.Th).

**Variante 1b-4:** Verbindung **4** (28,0 g) wird in THF (80ml) suspendiert. Danach wird innerhalb von 2,5 Stunden ein Gemisch aus THF (200 mL) und Chloracetylchlorid **5b-4'** (10,0 g) bei 50°C zugeben und im Anschluss mit NaOH (2mol/l, 50ml) alkalisch gestellt. Danach wird die Wasserphase abgetrennt und die organische Phase eingeengt und in Butylacetat aufgenommen. Die Phasen werden wieder getrennt, die organische Phase im Vakuum eingeengt und bei 45°C mit MTBE (240 mL) gefällt. Es wird auf -2°C abgekühlt und filtriert. Das Produkt wird mit einem Gemisch aus Butylacetat und tert-Butylmethylether (MTBE) (50 mL) gewaschen. Der Filterkuchen wird im Vakuum getrocknet und man erhält 23,3 g Produkt **6** (71 % d.Th).

### STUFE 2: SYNTHESE VON ß-ALANIN-N-[[2[[[4[[[(HEXYLOXY)CARBONYL]4AMINO]-IMINOMETHYL]PHENYL]AMINO]METHYL]-1-METHYL-1 H-BENZIMIDAZOL-5YL]CARBONYL]-N-2-PYRIDINYL-ETHYL ESTER

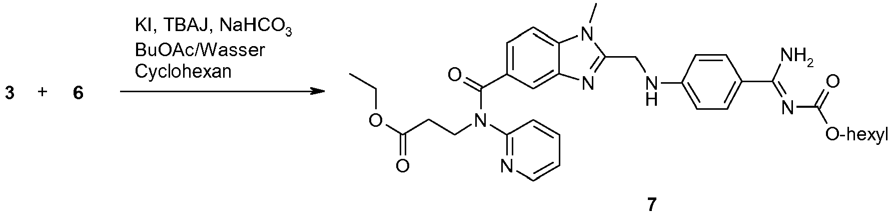

Verbindung **3** (7,7 g) wird in Butylacetat (65 mL), Natronlauge (25 mL, 45%ig) und Wasser (25 mL) vorgelegt und auf 50°C erwärmt. Danach werden die Phasen getrennt und die org. Phasen nochmals mit Wasser (30 mL) extrahiert. Die organische Phase wird mit Natriumiodid (1,54 g), Natriumhydrogencarbonat (4,00 g), Tetrabutylammoniumiodid (0,75 g), Verbindung **6** (10,0 g), Cyclohexan (65 mL) und Wasser (30 mL) versetzt und 2h bei 40°C gerührt. Dann wird unter Vakuum das Cyclohexan abdestilliert, Butylacetat (95 mL) zugeben und 2h bei 70°C gerührt. Im Anschluss werden die Phasen getrennt und die organische Phase zweimal mit Wasser (10 mL) extrahiert. Die organische Phase wird unter Vakuum eingeengt, die Lösung auf 0°C abgekühlt und filtriert. Das Produkt wird mit Butylacetat (30 mL) gewaschen. Der Filterkuchen wird im Vakuum getrocknet und man erhält 13,8 g Produkt **7** (87,8 % d.Th).

### STUFE 3: ß-ALANIN-N-[[2-[[[4-[[[ (HEXYLOXY)-CARBONYL]4-AMINO]IMINO-METHYL]PHENYL]AMINO] METHYL]-1-METHYL-1 H-BENZIMIDAZOL-5YL]CARBONYL]-N-2-PYRIDINYL-ETHYL ESTER-METHANESULFONAT.

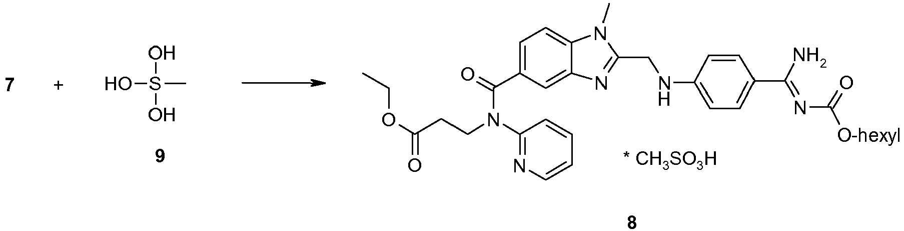

Verbindung **7** (20 g) wird in Aceton (238 mL) bei Raumtemperatur suspendiert und auf Rückfluss erhitzt. Die Lösung wird klärfiltriert und mit Aceton (20 mL) nachgespült. Das Filtrat wird auf 33 °C abgekühlt und eine auf 0°C gekühlte Lösung aus Methansulfonsäure (3,0 g) in Aceton (34 mL) zudosiert und mit Aceton (5,0 mL) nachgespült. Dann wird auf 20 °C gekühlt filtriert. Das Produkt wird mit Aceton (54 mL) gewaschen. Der Filterkuchen wird im Vakuum getrocknet und man erhält 22,2 g Produkt **8** (96,3 % d.Th).

## Patentansprüche

1. Verfahren zur Herstellung von Verbindungen der Formel **7**, 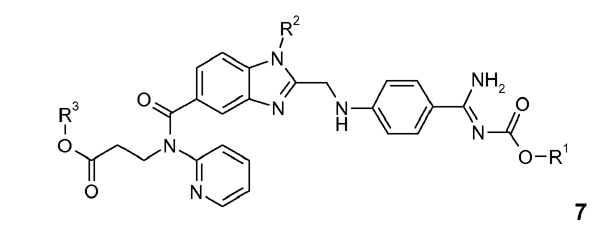 **dadurch gekennzeichnet, dass** eine Verbindung der Formel **6** 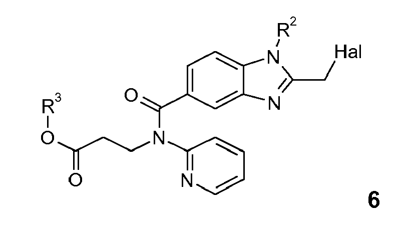 mit einer Verbindung der Formel **3** 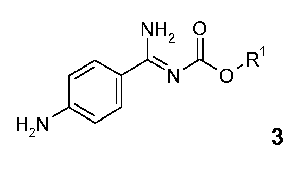 umgesetzt wird, worin in den obigen Verbindungen 7, **6** und **3** die Reste R¹, R² und R³ jeweils unabhängig voneinander C₁₋₆-alkyl und Hal = Chlor oder Brom bedeuten.

2. Verfahren nach Anspruch 1, worin die Verbindung der Formel **6** 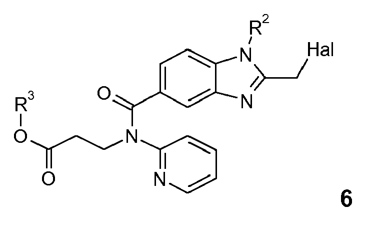 durch Reaktion einer Verbindung der Formel **4** 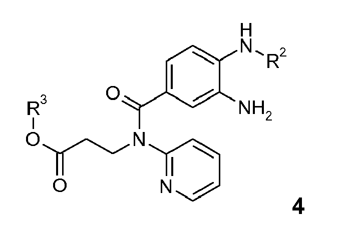 mit einer Verbindung der Formel **5,** ausgewählt aus der Gruppe bestehend aus Halogenessigsäureanhydrid **5b-1,** Halogenessigsäure **5b-2,** Halogenessigsäureorthoester **5b-3** oder Halogenacetylchlorid **5b-4,** worin Halogen (Hal) für Chlor oder Brom stehen kann, hergestellt wird, worin in den obigen Verbindungen **4** und **6** die Reste R¹, R² und R³ jeweils unabhängig voneinander C₁₋₆-alkyl bedeuten.

3. Verfahren nach Anspruch 1 oder 2, worin die Verbindung der Formel **3** durch Reaktion einer Verbindung der Formel **1** 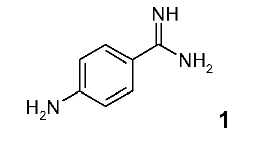 mit einer Verbindung der Formel **2** 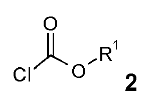 hergestellt wird, worin in den obigen Verbindungen **3** und **2** die Reste R¹, R² und R³ jeweils unabhängig voneinander C₁₋₆-alkyl bedeuten.

4. Verfahren nach einem der Ansprüche 1 bis 3, worin die Reste R¹, R² und R³ jeweils unabhängig voneinander Methyl, Ethyl, Propyl, Butyl oder Hexyl bedeuten.

5. Verfahren nach einem der Ansprüche 1 bis 4, worin die Reste R¹ = Hexyl; R² = Methyl und R³ = Ethyl bedeuten.

6. Verfahren nach einem der Ansprüche 1 bis 5, worin die Verbindung der Formel **5** ausgewählt ist aus der Gruppe bestehend aus Chloressigsäureanhydrid **5b-1',** Chloressigsäure **5b-2',** Chloressigsäureorthoester **5b-3'** oder Chloracetylchlorid **5b-4'.**

7. Verfahren nach einem der Ansprüche 1 bis 5, worin die Verbindung der Formel **5** ausgewählt ist aus der Gruppe bestehend aus Chloressigsäureanhydrid **5b-1'** oder Chloressigsäureorthoester **5b-3'.**

8. Verfahren nach einem der Ansprüche 1 bis 7 worin die Umsetzung der Intermediats **6** mit einer Verbindung der Formel **3** in einem zweiphasigen Lösungsmittelsystem bestehend aus Wasser und einem damit nicht mischbaren, organischen Lösungsmittel stattfindet.

9. Verfahren nach Anspruch 8 worin die Umsetzung der Intermediats **6** mit einer Verbindung der Formel **3** in einem zweiphasigen Lösungsmittelsystem bestehend aus Wasser und einem oder mehreren Lösungsmitteln ausgewählt aus der Gruppe bestehend aus Toluol, Tetrahydrofuran, 2-Methyltetrahydrofuran, Butylacetat, Cyclohexan und Ethylacetat stattfindet.

10. Verfahren nach einem der Ansprüche 1 bis 9 worin die Verbindung **7** mit Methansulfonsäure **9** zu einer Verbindung der Formel **8** 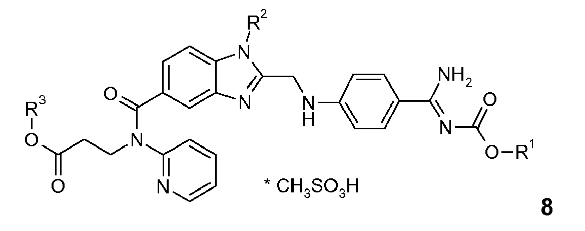 umgesetzt wird.

11. Verbindung der Formel **6** 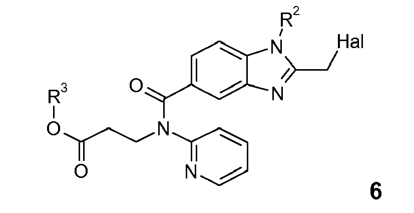 worin die Reste R² und R³ jeweils unabhängig voneinander C₁₋₆-alkyl und Hal = Chlor oder Brom bedeuten.

12. Verbindung nach Anspruch 11, worin R² = Methyl, R³ = Ethyl und Hal = Chlor bedeutet.

## Claims

1. Process for preparing compounds of formula **7,** 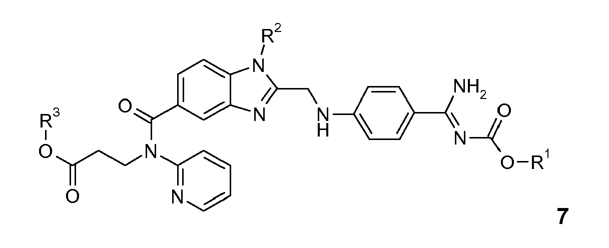 **characterised in that** a compound of formula **6** 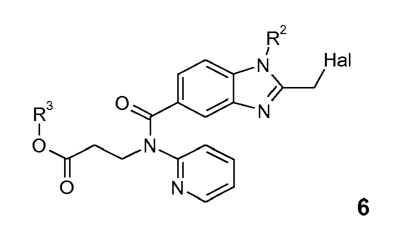 is reacted with a compound of formula **3** 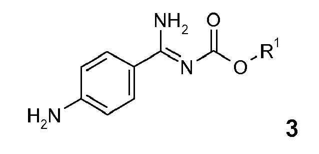 wherein in the above compounds **7, 6** and **3** the groups R¹, R² and R³ each independently of one another denote C₁₋₆-alkyl and Hal = chlorine or bromine.

2. Process according to claim 1, wherein the compound of formula **6** 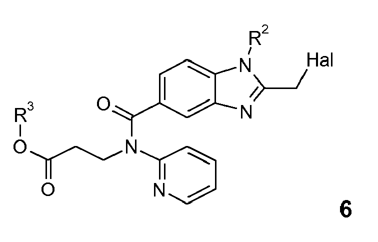 is prepared by reacting a compound of formula **4** 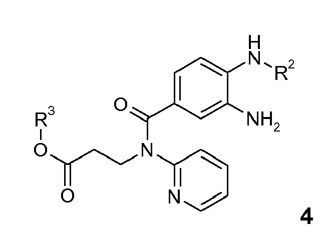 with a compound of formula **5,** selected from among haloacetic acid anhydride **5b-1,** haloacetic acid **5b-2,** ortho-haloacetate **5b-3** or haloacetyl chloride **5b-4,** wherein halogen (Hal) may denote chlorine or bromine, while in the above compounds **4** and **6** the groups R¹, R² and R³ each independently of one another denote C₁₋₆-alkyl.

3. Process according to claim 1 or 2, wherein the compound of formula **3** is prepared by reacting a compound of formula **1** 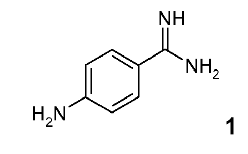 with a compound of formula **2** 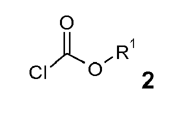 wherein in the above compounds **3** and **2** the groups R¹, R² and R³ each independently of one another denote C₁₋₆-alkyl.

4. Process according to one of claims 1 to 3, wherein the groups R¹, R² and R³ each independently of one another represent methyl, ethyl, propyl, butyl or hexyl.

5. Process according to one of claims 1 to 4, wherein the groups R¹ = hexyl; R² = methyl and R³ = ethyl.

6. Process according to one of claims 1 to 5, wherein the compound of formula **5** is selected from among chloroacetic acid anhydride **5b-1',** chloroacetic acid **5b-2',** chloroacetic ortho ester **5b-3'** or chloroacetyl chloride **5b-4'.**

7. Process according to one of claims 1 to 5, wherein the compound of formula **5** is selected from among chloroacetic acid anhydride **5b-1'** or chloroacetic ortho ester **5b-3'.**

8. Process according to one of claims 1 to 7, wherein the reaction of the intermediate **6** is carried out with a compound of formula **3** in a two-phase solvent system consisting of water and an organic solvent which is immiscible therewith.

9. Process according to claim 8, wherein the reaction of the intermediate **6** is carried out with a compound of formula **3** in a two-phase solvent system consisting of water and one or more solvents selected from among toluene, tetrahydrofuran, 2-methyltetrahydrofuran, butyl acetate, cyclohexane and ethyl acetate.

10. Process according to one of claims 1 to 9, wherein the compound **7** is reacted with methanesulphonic acid **9** to form a compound of formula **8**

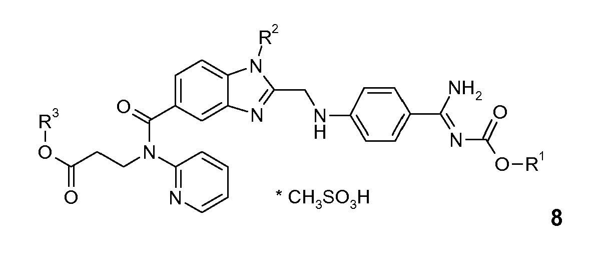

11. Compound of formula **6** 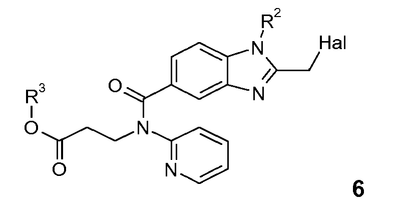 wherein the groups R² and R³ each independently of one another denote C₁₋₆-alkyl and Hal = chlorine or bromine.

12. Compound according to claim 11, wherein R² = methyl, R³ = ethyl and Hal = chlorine.

## Revendications

1. Procédé de production de composés de formule 7 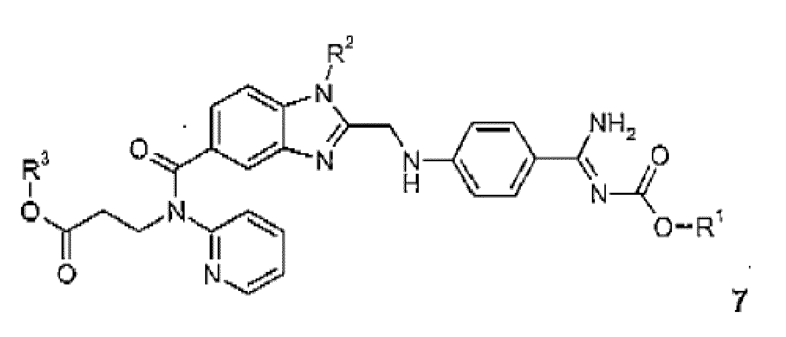 **caractérisé en ce que** l'on fait réagir un composé de formule 6 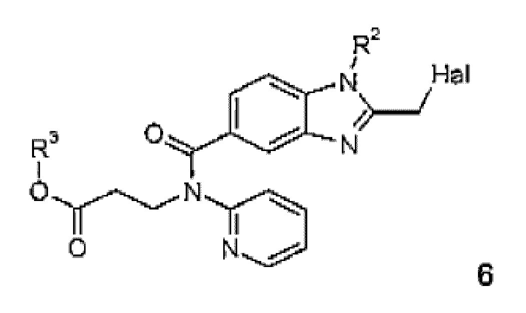 avec un composé de formule 3 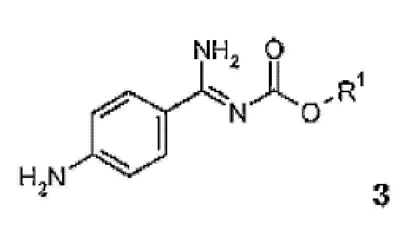 dans laquelle , dans les composés 7, 6 et 3 ci-dessus, les radicaux R¹, R² et R³ désignent respectivement, indépendamment les uns des autres, un groupe alkyle en C₁ à C₆ et Hal = un atome de chlore ou de brome.

2. Procédé selon la revendication 1, dans lequel le composé de formule 6 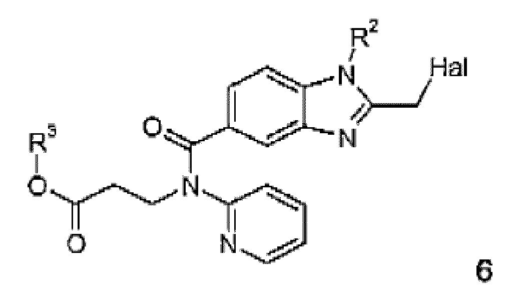 est produit par la réaction d'un composé de formule 4 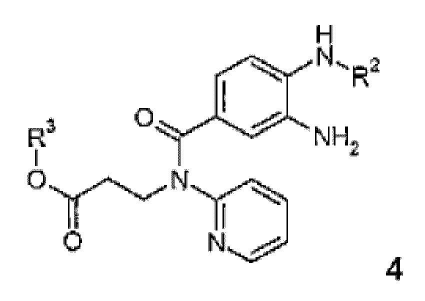 avec un composé de formule 5 choisi dans le groupe consistant en anhydride d'acide halogénoacétique 5b-1, en acide halogénoacétique 5b-2, en orthoester d'acide halogénoacétique 5b-3 ou en chlorure d'halogénoacétyle 5b-4, dans laquelle l'halogène (Hal) peut désigner un atome de chlore ou de brome, dans laquelle dans les composés 4 et 6 ci-dessus, les radicaux R¹, R² et R³ désignent respectivement, indépendamment les uns des autres, un groupe alkyle en C₁ à C₆.

3. Procédé selon la revendication 1 ou 2, dans lequel le composé de formule 3 est produit par la réaction d'un composé de formule 1 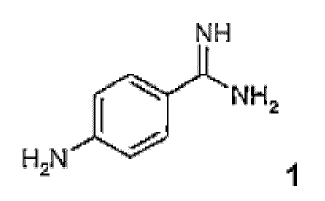 avec un composé de formule 2 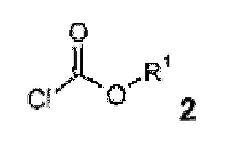 dans laquelle dans les composés 3 et 2 ci-dessus, les radicaux R¹, R² et R³ désignent respectivement, indépendamment les uns des autres, un groupe alkyle en C₁ à C₆.

4. Procédé selon l'une des revendications 1 à 3, dans lequel les radicaux R¹, R² et R³ désignent respectivement, indépendamment les uns des autres, un groupe méthyle, éthyle, propyle, butyle ou hexyle.

5. Procédé selon l'une des revendications 1 à 4, dans lequel les radicaux R¹ = un groupe hexyle, R² = un groupe méthyle et R³ = un groupe éthyle.

6. Procédé selon l'une des revendications 1 à 5, dans lequel le composé de formule 5 est choisi dans le groupe consistant en anhydride d'acide chloroacétique 5b-1', en acide chloroacétique 5b-2', en orthoester d'acide chloroacétique 5b-3' ou en chlorure de chloroacétyle 5b-4'.

7. Procédé selon l'une des revendications 1 à 5, dans lequel le composé de formule 5 est choisi dans le groupe consistant en anhydride d'acide chloroacétique 5b-1' ou en orthoester d'acide chloroacétique 5b-3'.

8. Procédé selon l'une des revendications 1 à 7, dans lequel la réaction de l'intermédiaire 6 s'effectue avec un composé de formule 3 dans un système de solvant biphasique constitué d'eau et d'un solvant organique non miscible avec celle-ci.

9. Procédé selon la revendication 8, dans lequel la réaction de l'intermédiaire 6 s'effectue avec un composé de formule 3 dans un système de solvant biphasique constitué d'eau et d'un ou plusieurs solvants choisis dans le groupe consistant en toluène, tétrahydrofurane, 2-méthyltétrahydrofurane, acétate de butyle, cyclohexane et acétate d'éthyle.

10. Procédé selon l'une des revendications 1 à 9, dans lequel on fait réagir le composé 7 avec de l'acide méthanesulfonique 9 en un composé de formule 8

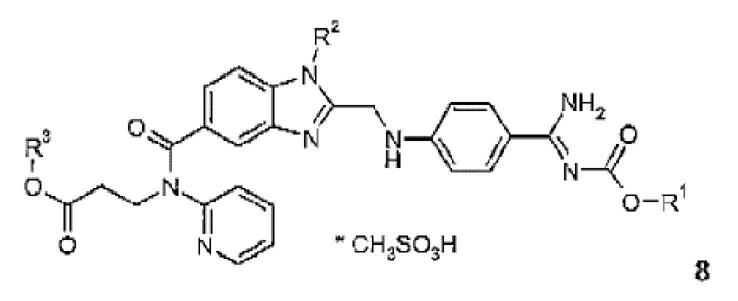

11. Composé de formule 6 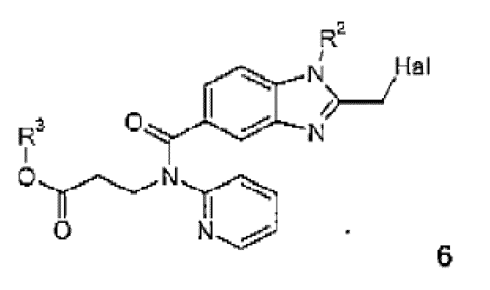 dans laquelle les radicaux R² et R³ désignent respectivement, indépendamment les uns des autres, un groupe alkyle en C₁ à C₆ et Hal = un atome de chlore ou de brome.

12. Composé selon la revendication 11, dans lequel R² = un groupe méthyle, R³ = un groupe éthyle et Hal = un atome de chlore.
